# EUROPÄISCHE PATENTSCHRIFT

(11) **EP 2 552 342 B1**
(45) Veröffentlichungstag und Bekanntmachung des Hinweises auf die Patenterteilung: **17.09.2014**
(21) Anmeldenummer: 11711522.0
(22) Anmeldetag: 28.03.2011
(51) Int. Cl.: A61C 11/00, A61C 13/00

(54) **VERFAHREN UND ANORDNUNG ZUM BILDEN EINES DENTALMODELLS**
METHOD AND ARRANGEMENT FOR FORMING A DENTAL MODEL
PROCÉDÉ ET DISPOSITIF DE FORMATION D'UN MODÈLE DENTAIRE

(30) Priorität: 16.07.2010 DE 102010036436; 11.05.2010 DE 102010016882; 29.03.2010 DE 102010016199
(43) Veröffentlichungstag der Anmeldung: 06.02.2013
(73) Patentinhaber: DeguDent GmbH, 63457 Hanau (DE)
(72) Erfinder: ERTL, Thomas, 61197 Florstadt (DE)
(74) Vertreter: Stoffregen, Hans-Herbert
(86) Internationale Anmeldenummer: PCT/EP2011/054678
(87) Internationale Veröffentlichungsnummer: WO 2011/120893

(56) Entgegenhaltungen:
- WO-A1-2004/030565
- CH-A- 315 737
- NL-A- 7 706 907
- US-A- 4 276 022

## Beschreibung

Die Erfindung bezieht sich auf ein Verfahren zur Bildung eines physischen oder virtuellen Dentalmodells als Hilfsmittel für die Herstellung von Zahnersatz sowie dessen Herstellung, wobei Form zumindest eines in einem Ober- oder Unterkiefer vorhandenen Zahnstumpfs, Kontaktpunkte zu zu dem zumindest einen Zahnstumpf benachbarten Zähnen sowie Okklusalfläche zumindest eines Gegenzahns im Bereich des Bewegungsfeldes des auf dem zumindest einen Zahnstumpf anzuordnenden Zahnersatzes unter Verwendung digitaler Daten des berührungslos gemessenen Ober- und Unterkiefers berücksichtigt werden.

Es sind eine Vielzahl von Anordnungen und Verfahren zur Herstellung von Zahnersatz bekannt. Im Allgemeinen wird nach der zahnärztlichen Präparation ein Abdruck des den Zahnersatz aufnehmenden Zahnstumpfes, dessen Umgebung und des Kiefers angefertigt. Gleiches gilt, wenn mehrere Zahnstümpfe mit einem Zahnersatz zu versehen sind. Unabhängig hiervon erfolgt dies üblicherweise mit Silikon-Vergussmasse, gleichwenn auch andere Materialien Verwendung finden können.

Aus dem Abdruck, der diese Situation im Mund des Patienten negativ darstellt, wird über eine Gipsabformung ein sogenanntes Meistermodell hergestellt. Dieses Modell zeigt die Situation im Mund des Patienten positiv. Mit diesem Modell modelliert der Zahntechniker mit seiner handwerklichen Fähigkeit ein Modell des Grundgerüstes des Zahnersatzes aus Wachs oder aus bei niedriger Temperatur schmelzendem und polymerisierend aushärtendem Kunststoff. Ein so hergestelltes Positiv-Modell dient sodann üblicherweise als Grundlage für den Zahnersatz.

Heutzutage wird jedoch das alte Gipsmodell mit neuer Technologie (Stereolithographie oder Fräsen) und neuen Materialien (Kunststoff) aus Stereolithographiedatensätzen der Okklusionsstruktur in Verbindung mit einer digitalen Sockelrepräsentation hergestellt. Dabei ist z.B. das Herausnehmen der präparierten Zahnstümpfe aus dem restlichen Modell vorgesehen und mit verschiedenen Verbindungsstrukturen realisiert. Auch ist eine rein statische Verschlussbisslage durch Verschlüsselung von Oberkiefer- und Unterkiefermodell mittels Verbindungsstrukturen verfügbar.

Nachteil der derzeit verfügbaren Realisierung ist der hohe Materialbedarf, der bei Gipsmodellen kein Problem darstellt, jedoch bei den teuren Kunststoffmaterialien, die für die Stereolithographie oder vergleichbare Verfahren notwendig sind, zu Kostennachteilen führt, die eine Wettbewerbsfähigkeit einschränken. Hinzu kommt die relativ lange Fertigungsdauer von mehreren Stunden, die in Verbindung mit hohen Investitionskosten von Rapid-Prototyping-Maschinen ebenfalls zu einer hohen Kostenbelastung führt.

Die DE-B-10 2005 033 738 bezieht sich auf ein Verfahren und Vorrichtung zur Herstellung von Zahnersatz. Dabei werden vorliegende Konstruktionsdaten mit Vermessungsdaten eines bearbeiteten herzustellenden Zahnersatzes auf einer Anzeige dargestellt.

Gegenstand der DE-A-10 2006 026 776 ist ein Verfahren zur Herstellung einer Zahnprothese, wobei ein digitales Erfassen einer Kiefersituation und -relation erfolgt.

Nach der DE-A-103 04 757 werden bei der Herstellung von Zahnersatz Kieferdaten berücksichtigt, die normalerweise an Patienten zur Einstellung eines Artikulators genommen werden. Ferner werden Kieferbewegungen in einem Rechner simuliert.

Das Herstellen von Zahnersatz unter Verwendung virtueller Prototypen eines Zahnersatzes ist der EP-A-1 935 369 zu entnehmen. Dabei wird ein Kieferbereich gescannt, in den ein Zahnersatz einzusetzen ist.

Die WO-A-2008/051130 bezieht sich auf ein Verfahren und eine Vorrichtung zur Herstellung von einem Dentalmodell unter Verwendung von Abdrücken.

Aus der WO-A-2008/030965 ist ein Verfahren zur Herstellung eines Zahnersatzes bekannt, bei dem ein virtuelles Ober- und Unterkiefermodell verwendet wird.

Die Herstellung eines Zahnersatzes unter Verwendung einer virtuellen Prothese wird in der WO-A-03/017864 beschrieben, wobei 3D-Daten eines Ober- und Unterkiefers aufgenommen werden, in den ein Zahnersatz einzusetzen ist.

Die US-A-2005/0070782 beschreibt die Herstellung von Zahnersatz unter Berücksichtigung virtueller Modelle. Hierzu wird auch ein virtueller Artikulator eingesetzt.

Der WO-A-2004/030565 ist ein Verfahren zur Bildung eines Dentalmodells als Hilfsmittel für die Herstellung von Zahnersatz sowie die Herstellung des Zahnersatzes selbst zu entnehmen. Dabei wird ein Negativmodell des Ober- und Unterkiefers benutzt, um ein virtuelles Dentalmodell zu generieren.

Aus der NL-A-7706907 ist ein Artikulator mit drei Abstützpunkten bekannt.

Die US-A-4 276 022 bezieht sich auf einen Artikulator mit einem Unter- und Oberteil, wobei das Oberteil über drei Punkte in Ausnehmungen des Unterteils abgestützt ist. In die Ausnehmungen kann ein Acrylmaterial eingebracht werden.

Gegenstand der CH-A-315 737 ist ein Artikulator mit Unter- und Oberteil, die über drei Punkte gegeneinander abgestützt sind.

Der vorliegenden Erfindung liegt die Aufgabe zugrunde, ein Verfahren und eine Anordnung der eingangs genannten Art derart weiterzubilden bzw. zur Verfügung zu stellen, das bzw. die eine kostengünstige Produktion wie Rapid-Prototyping-Produktion ermöglicht, wobei gleichzeitig von Patienten gewinnbare diagnostische Informationen in den Fertigungsprozess einfließen sollen.

Verfahrensmäßig wird die Aufgabe im Wesentlichen dadurch gelöst, dass zumindest nachfolgende Verfahrensschritte berücksichtigt werden:
a) Aufnehmen des Ober- und Unterkiefers zur Ermittlung erster Daten,
b) Aufnehmen des Bewegungsfeldes des Kiefergelenks in Disklusion zur Ermittlung zweiter Daten,
c) Aufnehmen des Kontaktfeldes der Zähne in Okklusion zur Ermittlung dritter Daten,
d) Berechnung von Bewegungsbahnen zwischen Unter- und Oberkiefer aus zumindest den ersten Daten und den dritten Daten oder den ersten und den zweiten Daten oder den zweiten und den dritten Daten oder den ersten und den zweiten und den dritten Daten,
e) Verwenden einer Halteeinrichtung mit Unterteil und zu diesem verstellbarem Oberteil, wobei mit dem Oberteil ein den Oberkiefer repräsentierendes erstes Element mit ersten Aufnahmen und mit dem Unterteil ein den Unterkiefer repräsentierendes zweites Element mit zweiten Aufnahmen verbunden ist oder wird,
f) Herstellen von drei Abstützpunkten oder -flächen, die von den gemäß Schritt d) berechneten Bewegungsbahnen gebildet werden und die in ortsfester Beziehung zu dem Unterteil angeordnet werden oder sind und auf denen sich das Oberteil über Abstützelemente abstützt,
g) Herstellen des zumindest einen Stumpfs sowie zumindest einer zugewandten Seite der benachbarten Zähne sowie der Okklusionsfläche des zumindest einen Gegenzahns und Anordnung dieser in den ersten und zweiten Aufnahmen unter Berücksichtigung der ersten, zweiten und dritten Daten und
h) Herstellen des Zahnersatzes auf dem zumindest einen Stumpf,
wobei die Verfahrensschritte b), c) alternativ oder sowohl der Verfahrensschritt b) als auch der Verfahrensschritt c) durchgeführt werden.

Die Verfahrensschritte e), f), g) und h) können virtuell durchgeführt werden.

Hinsichtlich des Verfahrensschritts c) sollte die Okklusion in Zentrik und umfänglichem Bewegungsfeld aufgenommen werden. Ferner besteht die Möglichkeit, dass die Okklusion im Schritt c) mit therapeutischer Korrektur aufgenommen wird. Auch besteht die Möglichkeit, die Okklusion im Verfahrensschritt c) zwangsgeführt durchzuführen.

In Weiterbildung der Erfindung ist vorgesehen, dass das Resilienzfeld des Kiefergelenks und/oder Mandibula und/oder Zahnapparat zur Ermittlung vierter Daten aufgenommen wird, wobei die Abstützpunkte oder -flächen unter Berücksichtigung der vierten Daten in ihrer Resilienz ausgebildet werden.

Des Weiteren sieht eine Ausgestaltung vor, dass den Abstützungen Bewegungsbegrenzunaselemente zugeordnet werden oder sind, durch die die Bewegung des Oberteils zu dem Unterteil entsprechend der Kieferbewegung und/oder des gemessenen Resilienzfeldes des Kiefergelenks begrenzt wird, wobei insbesondere die Kieferbewegung zumindest entsprechend Verfahrensschritt b) ermittelt wird.

Ferner kann ergänzend die Resilienz des Zahnhalteapparates von zumindest einem Zahn zur Ermittlung fünfter Daten gemessen werden, die zumindest beim Verfahrensschritt d) berücksichtigt werden.

Unabhängig hiervon können anstelle des Verfahrensschrittes c) laterale Bewegungen des Unterkiefers zum Oberkiefer unter Verwendung eines Verfahrens der minimalen Zunahme potentieller Energie bei den Bewegungen zur Ermittlung sechster Daten berechnet werden, die die dritten Daten ersetzen.

Die Erfindung zeichnet sich auch dadurch aus, dass die aus zumindest den ersten und dritten Daten oder alternativ sechsten Daten erfolgende Berechnung der Abstützpunkte oder -flächen gemäß Verfahrensschritt f) unter Reduktion der Freiheitsgrade der Beweglichkeit des Unterkiefers relativ zum Oberkiefer durchgerührt werden, wobei eine Rotation um die Sagittalachse (x-Achse), eine Rotation um die Transversalachse (y-Achse) und eine Translation entlang der z-Achse senkrecht zur Kauebene ausgeschlossen werden.

Als Ausgangspunkt für die Berechnung der lateralen Bewegung kann Zentrik von Oberkiefer und Unterkiefer gewählt werden.

Zur Ermittlung der Zentrik sollte von Unterkiefer und Oberkiefer buccale Seitenfläche der Zähne des Oberkiefers und Unterkiefers in Schlussbissstellung gescannt werden.

Um die ersten, zweiten und dritten Daten zu erhalten, werden diese intraoral ermittelt.

Erfindungsgemäß ist es möglich, dass nicht nur eine physische Halteeinrichtung und ein reales Herstellen vom Stumpf in oraler Umgebung, also der benachbarten Zähnen und einen oder mehreren Antagonisten bzw. Teilen dieser, hergestellt werden, sondern dass die für die Herstellung des Zahnersatzes erforderlichen Kieferbereiche virtuell erzeugt und virtuell der Zahnersatz unter Berücksichtigung der durch Scannen des Ober- und Unterkiefers ermittelten Daten hergestellt und sodann die dem virtuellen Zahnersatz entsprechenden Daten benutzt werden, um im CAM-Verfahren den Zahnersatz herzustellen, wie dies bei bekannten CAD-/CAM-Verfahren ermöglicht wird.

Dabei besteht erfindungsgemäß auch die Möglichkeit, Elastizitätsmodule der einzelnen anatomischen Strukturen nachzubilden, die beispielsweise durch die elastische Aufhängung der Zähne durch den Zahnhalteapparat bedingt sind. Auch kann das Elastizitätsmodul des Kiefergelenks berücksichtigt werden. Somit werden Fehler in der Bestimmung der Okklusal- und Interproximalkontakte vermieden.

In Abhängigkeit vom Umfang der Datengewinnung am Patienten sind erfindungsgemäß verschiedene Vorgehensweisen möglich.

So kann zunächst mittels Scannen oder einem gleichwertigen berührungslosen Verfahren, wie einem unter der Bezeichnung Zcbris angebotenen System, eine Aufnahme des Kiefergelenkbewegungsfeldes in Disklusion, also Ermittlung des verfügbaren Bewegungsvolumens bei fehlender Okklusion erfolgen. Das Bewegungsfeld kann dabei unforciert und forciert ermittelt werden.

Das unforcierte Kennfeld des verfügbaren Bewegungsvolumens ist dabei durch die anatomischen und pathologischen Begrenzungen der beiden Kiefergelenke sowohl durch die Gelenkbahnen als auch durch die ligamentären Begrenzungen definiert. Bei der unforcierten Kennfeldbestimmung führt der Patient selbst freie Kieferbewegungen in Nichtkontakt, also Disklusion durch, und zwar sowohl Grenzbewegungen als auch Bewegungen im Volumen, um den Bereich des Kennfeldes innerhalb der Bewegungsgrenzen zu füllen.

Die aktuelle muskulär definierte Ruheschwebelage wird durch eine Datenaufzeichnung über einen Zeitraum erreicht, in dem sich die Abweichungen in einer Messdatenfolge stabilisieren.

Ergänzend oder alternativ kann eine forcierte Bewegung erfolgen. Hierbei wird der Unterkiefer durch einen Zahnarzt geführt. Dies ist zur Ermittlung hinreichend notwendiger Daten zur Ermittlung des Kiefergelenkbewegungsfeldes häufig erforderlich, da viele Patienten nicht in der Lage sind, Schlussbiss- oder Exkursionsbewegungen ohne Hilfestellung korrekt auszuführen. Wird der Unterkiefer moderat geführt, kann die Stellung der Bewegung durch die Einwirkung des Zahnarztes akzeptabel sein.

Aus den unterschiedlichen Messungen, also der Differenz zwischen den Bewegungsfeldern der forcierten und unforcierten Vorgehensweise können zusätzlich Rückschlüsse auf muskuläre Fehlfunktionen erzielt werden.

Auch bei der Findung der Ruheschwebelage kann ein moderates Führen in eine retrale Kontaktposition oder eine zu dieser nahen Position sinnvoll sein.

Unabhängig hiervon sind auf jeden Fall digitale 3D-Daten des Unter- und Oberkiefers zu ermitteln.

Sodann wird nach Ermittlung des Kiefergelenkbewegungsfeldes in Disklusion - sofern dieser Schritt durchgeführt wird - das Zahnkontaktfeld in Okklusion ermittelt, wobei der Patient den Unterkiefer mit minimaler Kaukraft in beliebigen Bahnen in Okklusion gegenüber dem Oberkiefer bewegt, so dass der gesamte Okklusionsbereich erfasst wird. Die Okklusionscharakteristiken werden somit als Kennfelddaten abgespeichert. Die diesbezügliche Ermittlung des Zahnkontaktfeldes in Okklusion umfasst folglich Ober- und Unterkiefer in Zentrik und um die Zentrik sich erstreckendes Bewegungsfeld.

Gegebenenfalls kann auch eine Aufnahme des Bewegungsfelds mit therapeutischer Korrektur durchgeführt werden, d.h. eine Bisslagejustierung z. B. mittels Aufbissschiene in Vorbereitung einer Okklusionskorrektur bei einem phatologischen Okklusionsbefund. Eine therapeutische Korrektur der Okklusion kann aber auch mit eingesetzter Korrekturschiene erfasst werden, z. B. zur Vorbereitung einer Bisshebung. Interferenzen können durch einen Vergleich mit den Daten ohne Einsatz der Schiene ermittelt werden.

Anstelle des Kiefergelenkbewegungsfeldes in Okklusion besteht alternativ oder ergänzend auch die Möglichkeit, dass aus den in Zentrik von Ober- und Unterkiefer ermittelten Daten mögliche lateral verschobene Positionen des Unterkiefers relativ zum Oberkiefer zueinander berechnet werden, indem ein Verfahren des minimalen Zuwachses potentieller Energie angewendet wird. Dabei wird die Zunahme potentieller Energie bei einer infinitesimalen Bewegung in eine vorgegebene Richtung berechnet. Erwähntermaßen beginnt die Bewegung in der Zentrik, die als absolutes Minimum den kleinsten Abstand der Schwerpunkte von Oberkiefer und Unterkiefer darstellt. Die Aneinanderreihung solcher kleinster Bewegungsschritte mit minimalem Energiezuwachs ergeben die wahrscheinlichsten relativen Bewegungen, allerdings ohne den Einfluss der Kiefergelenke, was in unmittelbarer Umgebung um die Zentrik jedoch akzeptabel ist. Einschränkend auf die möglichen Bewegungsbahnen wirkt die ligamentäre, also die Bändchen- und Knorpelbegrenzung durch beide Kiefergelenke. Dies kann mittelwertig durch Annahme einer höchstwahrscheinlichen Lagebeziehung der Kondylen und der Zahnbögen angenommen werden und mittels Kieferregistrierung auch gemessen werden (Bewegungsfeld in Okklusion und in Disklusion).

Die Okklusionsgeometrie für Ober- und Unterkiefer wird unabhängig von dem angewendeten Verfahren, sei es Messen des Kiefergelenkbewegungsfeldes in Disklusion und Okklusion oder zur Ermittlung der Bewegungsbahnen nach dem Verfahren des minimalen Zuwachses der potentiellen Energie, mittels intraoralen Scannens ermittelt und liegt in digitaler Form vor. Dabei liegt aus den Daten der von buccal gescannten Seitenflächen der Zähne in Kontakt oder dem Scannen eines Wachsbisses die Relation von Unterkiefer und Oberkiefer zueinander in Zentrik vor.

Werden die Bewegungsfelder durch Messen ermittelt, so wird in weiterer hervorzuhebender und eigenerfinderischer Ausgestaltung ergänzend das Resilienzfeld berücksichtigt. Dabei kann das Resilienzfeld die Nachgiebigkeit von Kiefergelenk und Mandibula und ergänzend die Resilienz des Zahnhalteapparats von einem Zahn oder mehreren Zähnen berücksichtigen.

Zur Bestimmung der Kiefergelenks- und Mandibularesilienz führt der Patient die gleichen Bewegungen aus, wie bei der Aufnahme des Zahnkontaktfeldes in Okklusion, allerdings mit Kaukräften, die denen beim Kauen entsprechen. Um dies zu erreichen, lässt man den Patienten auf ein teilelastisches Medium wie Kaugummi kauen. Durch die hierdurch einwirkenden Kaukräfte wird entsprechend der jeweiligen Elastizitätsmodule der beteiligten anatomischen Strukturen das Okklusionssystem verformt. In erster Linie sind hierbei Kiefergelenk, Mandibula und Zahnhalteapparat von Relevanz.

Erwähntermaßen können diese Daten durch Messungen der Resilienz von anderen Zähnen ergänzt werden, die durch Kraft-Weg-Profile in eine Raumrichtung oder näherungsweise mittels Impulsantwortmessung bestimmt werden. Ein geeignetes Gerät hierfür ist das unter der Bezeichnung Periotest auf dem Markt erhältliche.

Die so gewonnenen Daten können mit den Daten, die durch die Aufnahme des Zahnkontaktfeldes in Okklusion ermittelt worden sind, in Beziehung gesetzt werden, um relevante Verformungen zu erkennen, die bei der Konstruktion des Zahnersatzes berücksichtigt werden.

Somit werden Möglichkeiten geboten, die der Stand der Technik nicht kennt, da weder die Kiefergelenkresilienz noch die Einzelzahnresilienz, also die Resilienz des Zahnhalteapparats nach den bekannten Verfahren vorgesehen noch eine Einbeziehung möglich ist. Hierdurch bedingt ein signifikanter Anteil an Informationen verloren gehen, der eine Optimierung des Zahnersatzes nicht zulässt und dazu führen kann, dass Einschleifmaßnahmen am Patienten erfolgen, um die durch die nicht zur Verfügung stehenden Daten bedingten Ungenauigkeiten auszugleichen.

Um das Kiefergelenkbewegungsfeld und das Zahnkontaktfeld in Okklusion oder Disklusion zu ermitteln, kann ein unter der Bezeichnung Zebris verfügbares System benutzt werden.

Hinsichtlich der Resilienzdaten, die das Kiefergelenk, die Mandibula und den Zahnhalteapparat betreffen, ist anzumerken, dass diese grundsätzlich nur integral über den gesamten Kiefer mit konventionellen Systemen gewonnen werden.

Soll mit Hilfe eines realen Modells Zahnersatz hergestellt werden, sollen also manuelle Arbeitsschritte eines Zahntechnikers erfolgen, muss in einer digitalen Prozesskette beginnend mit einer intraoralen digitalen 3D-Datengewinnung zuvor beschriebener Art zusätzlich ein reales Hilfsmittel zur Verfügung gestellt werden, das mindestens einer den derzeitigen Gipsmodellen vergleichbarer Funktionalität vorliegen.

Hierzu ist es zwingend erforderlich, dass nachstehende Funktionen und Eigenschaften bekannt sind:
- Form des präparierten Zahnstumpfes der präparierten Zahnstümpfe,
- Kontaktpunkte zu den Nachbarzähnen,
- Kauflächen der Gegenzähne im Bereich des Bewegungsfeldes des bzw. der präparierten Zähne,
- Möglichkeit Ober- und Unterkiefer in Zentrik zu stellen.

Erfindungsgemäß stehen des Weiteren Aussagen zur Verfügung über
- Dynamische Okklusion, entweder arbiträr (mittelwertig) oder individuell.

Ergänzend können aufgrund der erfindungsgemäßen Lehre Daten zur Verfügung gestellt werden betreffend
- Resilienzverhalten zumindest von einem der Elemente der Gruppe Kiefergelenk, Zahnaufhängung im Knochen, Mandibula und Zahnhalteapparat.

Ergänzend können Werte berücksichtigt werden betreffend
- Elastizitätsmodul des Knochens,
die aus der Literatur gewonnen werden.

Auch müssen die Achsen definiert werden, wobei üblicherweise festgelegt wird:
X-Achse: Saggitalachse
Y-Achse: Transversalachse

- Z-Achse: senkrecht zur Kauebene.

Um nach dem neuen Verfahren und unter Einsatz der neuen Materialien, wie diese eingangs beschrieben worden sind, kostenmäßig vertretbar einen Zahnersatz herstellen zu können, wobei zusätzlich bei der Ermittlung der Daten von dem konventionellen Verfahren der Abdrucknahme Abstand genommen wird und ein berührungsloses Scannen erfolgt, ist ein Kernmerkmal zur Kostenreduktion die Minimierung von notwendigem Bauraum, Material und Fertigungszeit. Unter Beibehaltung der notwendigen optionalen Eigenschaften ist dies durch Nutzung der zuvor erläuterten Datengewinnung am Patienten sowie die Berechnung der wahrscheinlichen Bewegungsbahnen möglich, wie dies zuvor beschrieben ist.

Entlang dieser Bewegungsbahnen werden durch Zahnkontakte des Unterkiefers relativ zum Oberkiefer bestimmte Bewegungsmöglichkeiten durch Reduktion der Freiheitsgrade verhindert (Rotation um die X-Achse und Y-Achse und Translation in Z-Achsenrichtung). Die Gleitbewegung des Unterkiefers im Zahnkontakt mit dem Oberkiefer, die dem gemessenen Zahnkontaktfeld in Okklusion entspricht, lässt sich somit durch drei Punkte des Koordinatensystems des Unterkiefers und des Koordinatensystems des Oberkiefers beschreiben.

Hierdurch wird man unabhängig von der durch Zahnkontakt erzwungenen Disklusion der Kieferbewegung aus der Zentrikposition heraus, da die notwendige Information in dem Kennfeld der Bewegung der Kiefer zueinander vorliegt, das das Zahnkontaktfeld in Okklusion - ohne zwingende Berücksichtigung des Resilienzfeldes - betrifft, inklusive der Information vom Kiefergelenk.

Hierdurch bedingt können theoretisch alle Zähne entfallen und ungeachtet dessen das Kennfeld der Bewegung im Zahnkontakt reproduziert werden, wenn die drei Punkte über jeweils eine Fläche geführt werden, die genau der entspricht, die die Punkte im Zahnkontakt beschrieben hätten.

Somit wird die Möglichkeit geschaffen, allein die Geometrieinformation, die zum Design des Zahnersatzes erforderlich sind, in einem physischen Modell zu verwenden, ohne auf die volle statische und dynamische Okklusionsinformation zu verzichten.

Praktisch wird dies durch drei Bewegungsfelder realisiert, die mit Gleitbahnen eines konventionellen Artikulators verglichen werden können, jedoch aufgrund der erfindungsgemäßen Lehre die vollständige Bewegungsinformation von Unterkiefer gegen Oberkiefer in Zahnkontakt enthalten.

Vereinfacht unter Inkaufnahme bestimmter Nachteile bestünde sogar die Möglichkeit, die drei Bewegungsfelder zu Punkten zusammenzuziehen, um sodann einen Zahnersatz zu konstruieren.

Unabhängig hiervon ist in Ausgestaltung der Erfindung vorgesehen, dass die Begrenzungsfunktion des Kiefergelenks einbezogen wird, und zwar durch so genannte Bewegungsbegrenzer, die den drei Punkte-Abstützungen zugeordnet sind.

Die Bewegungsfelder und Begrenzer sind individuelle Bauteile, die im Rapid-Prototyping-Verfahren gefertigt werden können. Aufnahmen zumindest der Bewegungsfelder sind in einer Halteeinrichtung vorgefertigt.

Um beispielsweise eine Einzelkrone zu fertigen, bedarf es daher lediglich des präparierten Zahnstumpfes, hinsichtlich der benachbarten beiden Zähne eines Ausschnitts der Interproximalflächen, die dem Zahnstumpf zugewandt sind, sowie eines Ausschnitts der Okklusalfläche der Gegenbezahnung, der den Bewegungsraum hinreichend abdeckt. Die Interproximalflächen und die Gegenokklusion können mit transparentem Material ausgeführt werden, um eine unmittelbare Beobachtung der Kontaktpunkte zu ermöglichen.

Die einzelnen Komponenten, also Zahnstumpf, Nachbarzähne bzw. deren Interproximalflächen aufweisende Abschnitte und Gegenbezahnung bzw. deren die Okklusalfläche aufweisende Abschnitte werden sodann entsprechend der ermittelten Daten einander zugeordnet. Hierzu wird bei der physischen Ausführung zur Ausbildung des Zahnersatzes an physischen Einzelkomponenten eine Haltereinrichtung mit Unterteil und zu diesem verstellbaren Oberteil verwendet, wobei mit dem Oberteil ein den Oberkiefer repräsentierendes erstes Element mit ersten Aufnahmen und mit dem Unterteil ein den Unterkiefer repräsentierendes zweites Element mit zweiten Aufnahmen verwendet wird. Die Beziehung der ersten und zweiten Elemente mit den ersten und zweiten Aufnahmen für die Einzelkomponenten wird aus den Scanndaten ermittelt.

Die Aufnahmen können als Pin/Lochmuster oder gekreuzte Streifenmuster auf Trägerplatten als die ersten und zweiten Elemente befestigt werden.

Somit kann der Materialverbrauch, die benötigte Baufläche und Bauhöhe für individuelle Bauteile minimiert werden.

Hervorzuheben ist außerdem, dass nach der erfindungsgemäßen Lehre, also den Einsatz minimierter individueller Bauteile, die Möglichkeit besteht, "voll rückwärts kompatibel" zu konventioneller Technik zu sein. Ausgehend von der beschriebenen Minimalkonfiguration ist eine Erweiterung um z. B. komplette Nachbarzähne oder komplette Gegenzähne bis hin zu zwei kompletten Kiefern möglich. In diesem Fall wird die Okklusionsinformation, die durch konventionelle Zähne geliefert wird, nicht in die Bewegungsfelder übernommen. Man erreicht somit die Skalierbarkeit von voller Abbildung des Kiefergelenks- und der Okklusionskennfelder in den drei Bewegungs- oder Gleitfeldern bis hin zu voll bezahnten Modellen, wobei lediglich die Kiefergelenksinformation über die Gleitfelder abgebildet wird.

So kann man z. B. einem Quadrantenmodell die volle Funktionalität eines vollen Modellpaares geben.

Zudem erhält man die Möglichkeit, die Bewegungsfelder derart auszubilden, dass die gemessene Resilienz nachempfunden wird.

Es kann somit die Resilienz in einem mechanischen Artikulatorsystem abgebildet werden. Dies kann dadurch realisiert werden, dass man die Bewegungsfelder aus einem Material herstellt, das der gemessenen Resilienz entspricht. Bevorzugterweise wird jedoch ein Bauteil als Bewegungsfeld benutzt, das aus zumindest zwei Teilen besteht, von denen eines eine hohe Elastizität aufweist. Durch die Variation der Schichtdicke kann die Resilienz individuell eingestellt werden, und zwar entsprechend der am Patienten gewonnenen Messdaten.

Aufgrund der erfindungsgemäßen Lehre wird ein physisches Modell zur Verfügung gestellt, das in seiner funktionalen Nutzbarkeit zumindest einem herkömmlichen Modell ebenbürtig ist und dabei nur jene Modellteile umfasst, die zur Herstellung des Zahnersatzes zwingend erforderlich sind. Die Einsparung an Bauzeit und Materialverbrauch ist besonders hervorzuheben, insbesondere dann, wenn die Herstellung nach Rapid-Prototyping-Verfahren erfolgt.

Die erfindungsgemäße Lehre lässt sich auch rein virtuell verwirklichen, so dass man entsprechend der zuvor beschriebenen Verfahrensweise einen Datensatz des Zahnersatzes erhält, der sodann im CAM-Verfahren hergestellt wird.

Die Erfindung zeichnet sich auch aus durch eine Anordnung zur Herstellung eines Zahnersatzes unter Berücksichtigung diagnostischer Informationen eines Patienten, für den der Zahnersatz herzustellen ist, umfassend eine Halterung mit einem Unterteil und einem zu diesem verstellbaren Oberteil, das über Abstützungen auf drei im Bereich von Ecken eines Dreiecks liegenden Abstützflächen gleitend abgestützt ist, die in ortsfester Beziehung zu dem Unterteil angeordnet sind oder von diesem ausgehen, wobei das Oberteil eine Halterung für ein einen Oberkiefer repräsentierendes erstes Element mit ersten Aufnahmen und das Unterteil eine Halterung für ein einen Unterkiefer repräsentierendes zweites Element mit zweiten Aufnahmen aufweist, wobei in den ersten und zweiten Aufnahmen zumindest ein mit dem Zahnersatz zu versehener Zahnstumpf, zumindest zugewandte Seiten von dem Zahnstumpf benachbarten Zähnen und Okklusalfläche zumindest eines Gegenzahns angeordnet sind, wobei die Abstützflächen Bewegungsinformationen von Unterkiefer und Oberkiefer in Zahnkontakt des Patienten enthalten und die Abstützflächen die Zuordnung der ersten und zweiten Aufnahmen und die Positionen des zumindest einen Zahnstumpfs sowie der zumindest zugewandten Seitenflächen und der Okklusalfläche auf der Basis von Daten berechnet sind, die mittels intraoraler Messung von Unter- und Oberkiefer des Patienten und zumindest der Stellung dieser in der Zentrik ermittelt werden, wobei die Abstützflächen Oberflächen eines mit dem Unterteil verbundenen Körpers sind, deren lokale Elastizitäten den auf die jeweiligen Abstützungspositionen umgerechneten Summenelastizitäten der Kiefergelenke und der Parodontien der jeweils im okklusalen Zahnkontakt stehenden Zahnpaare des Patienten entsprechen, und wobei zumindest zwei Abstützungen des Oberteils beabstandet zu den Abstützflächen von Begrenzungselementen umgeben sind, die die Bewegungsbegrenzung des Kiefers des Patienten berücksichtigen.

Dabei ist insbesondere vorgesehen, dass als Daten diejenigen benutzt werden, die nach dem erfindungsgemäßen Verfahren durch Aufnehmen des Ober- und Unterkiefers bzw. des Bewegungsfeldes des Kiefergelenkes in Disklusion bzw. des Kontaktfeldes der Zähne in Okklusion intraoral ermittelt werden, wobei die das Kontaktfeld der Zähne in Okklusion betreffenden Daten durch Daten ersetzt sein können, die durch laterale Bewegungen des Unterkiefers zum Oberkiefer unter Verwendung eines Verfahren der minimalen Zunahme potentieller Energie bei den Bewegungen berechnet werden.

Ferner ist vorgesehen, dass das Begrenzungselement eine die Resilienz des Kiefers berücksichtigende Elastizität aufweist.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung von der Zeichnung zu entnehmenden bevorzugten Ausführungsformen.

Es zeigen:
- Fig. 1: eine Anordnung zur Herstellung eines Zahnersatzes,
- Fig. 2: eine Prinzipdarstellung des erfindungsgemäßen Verfahrens,
- Fig. 3: eine Prinzipdarstellung von Einzelkomponenten zur Herstellung eines Zahnersatzes,
- Fig. 4a, 4b: Basisplatten zur Aufnahme der Einzelkomponenten und
- Fig. 5a, 5b: Prinzipdarstellungen von Bewegungsfeldern und Bewegungsbegrenzern.

Entsprechend der erfindungsgemäßen Lehre werden zur Herstellung eines Zahnersatzes intraoral Unter- und Oberkiefer eines Patienten gemessen, um digitale 3D-Daten zu erhalten. Sodann wird zumindest das Zahnkontaktfeld in Okklusion, d. h. in Zentrik von Unter- und Oberkiefer und umfängliches Bewegungsfeld benachbart der Zentrik ebenfalls vorzugsweise intraoral gemessen. Aus den buccal gescannten Seitenflächen der Zähne in Kontakt oder dem Scannen eines Wachsbisses, also einem zwischen dem aufeinanderliegenden Unter- und Oberkiefer angeordneten Element wie Silikonelement, ergibt sich die Relation von Unter- und Oberkiefer zueinander in Zentrik. Die diesbezüglichen Daten ggf. unter Berücksichtigung von Daten, die das Kiefergelenkbewegungsfeld in Disklusion sowie das Resilienzfeld bei forcierter Okklusion ggf. unter ergänzender Berücksichtigung von Einzelzahnresilienzen entsprechend zuvor erfolgter Erläuterungen betreffen, werden Bewegungsfelder durch Rapid-Prototyping-Verfahren hergestellt. Anstelle der Daten, die sich aus dem Zahnkontakt in Okklusion zuvor beschriebener Art unter Berücksichtigung des die Zentrik umgebenden Bewegungsfeldes ergeben, kann auch ein Verfahren des minimalen Zuwachses potentieller Energie ausgehend von Unter- und Oberkiefer in Zentrik benutzt werden.

Zur Herstellung des Zahnersatzes wird eine Anordnung 10 benutzt, die ein Oberteil 12 und ein Unterteil 14 umfasst, wobei das Oberteil 12 über drei Abstützungen 16, 18, 20 auf Bewegungsfeldern 22, 24, 26 gleitend abgestützt ist, die in ortsfester Position zum Unterteil 14 und insbesondere auf bzw. in diesem angeordnet sind. Die Abstützungen 16, 18, 20 weisen bevorzugt kugel- oder paraboloidförmige Enden auf, um im erforderlichen Umfang auf den Bewegungsfeldern 22, 24, 26 gleiten zu können.

An dem Oberteil 12 ist eine Aufnahmeplatte 28 für ein einen Oberkiefer repräsentierendes erstes Element 30 und an dem Unterteil 14 eine Aufnahmeplatte 32 für ein den Unterkiefer repräsentierendes zweites Element 34 befestigt.

Ferner sind zumindest zwei der Abstützungen 16, 18, 20, im Ausführungsbeispiel die Abstützung 18, 20, von Bewegungsbegrenzern 36, 38 umgeben, die die ligamentäre und knorpelgeführte Begrenzung des Kiefergelenks nachbilden.

Entsprechend der zuvor erläuterten erfindungsgemäßen Maßnahmen wird aus digitalisierten Messdaten durch Reduktion der Freiheitsgrade, also bevorzugterweise Ausschließen der Rotation des Unterkiefers um die Saggitalachse und Transversalachse und eine Translation in Richtung senkrecht zur Kauebene, die Gleitbewegung des Unterkiefers im Zahnkontakt mit dem Oberkiefer durch 3 Punkte des Koordinatensystems des Unterkiefers in dem Koordinatensystem des Oberkiefers beschrieben. Diese Bewegung wird durch die Möglichkeit des Gleitens des Oberteils 12 zu dem Unterteil 14 durch Abstützen der Abstützelemente 16, 18, 20 auf den auch als Abstützpunkte oderbereiche zu bezeichnenden Bewegungsfeldern 22, 24, 26 realisiert. Dabei liegen die Abstützpunkte auf den Kanten einer Dreiecksäule, so dass eine eindeutige Abstützung sichergestellt ist.

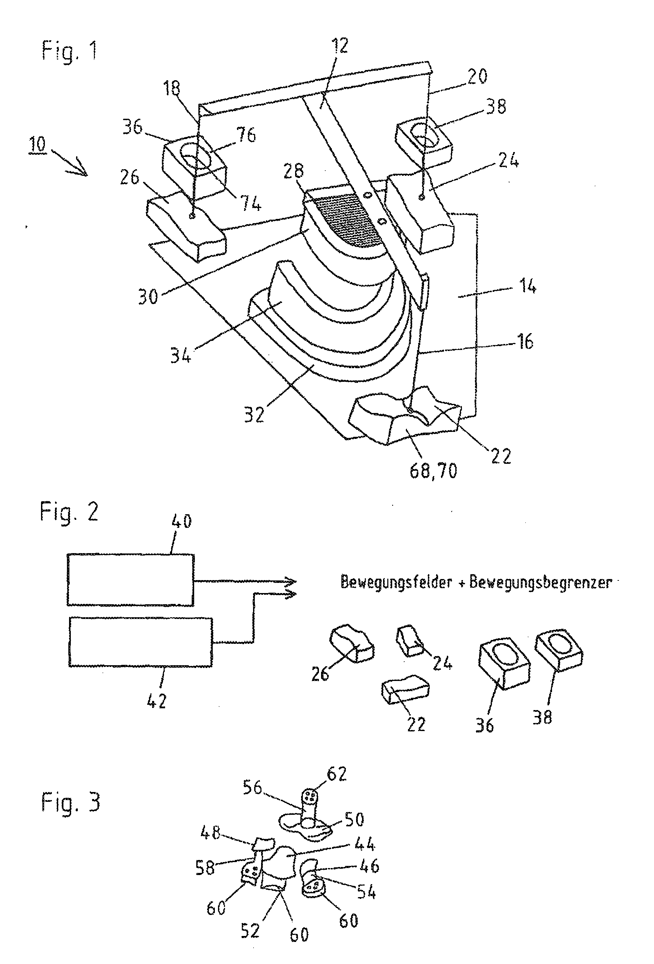

Der Fig. 2 ist prinzipiell zu entnehmen, dass aus der durch intraorales Scannen gemessenen Bewegungsanalyse des Unter- und Oberkiefers in zuvor beschriebener Weise (Daten 40), also zumindest unter Berücksichtigung der digitalen 3D-Daten des Ober- und Unterkiefers und der Okklusion in Zentrik und deren Umgebungsfeld, die Bewegungsfelder 22, 24, 26 sowie die Bewegungsbegrenzer 36, 38 berechnet und sodann aus den entsprechenden digitalen Daten mittels Rapid Prototyping Verfahrens die entsprechenden die Bewegungsfelder 22, 24, 26 aufweisenden Bauteile sowie die Bewegungsbegrenzer 36, 38 hergestellt werden.

Alternativ können die diesbezüglichen Bauteile auch auf der Basis der Daten ermittelt werden, die sich durch das Scannen des Unter- und Oberkiefers sowie der lateralen Bewegung von Unter- und Oberkiefer unter Berücksichtigung minimalen Zuwachses potentieller Energie bei Kenntnis der Zentrik ergeben (Daten 42).

Die zum Herstellen des Zahnersatzes benötigten Komponenten in individueller minimaler Ausführung sind der Fig. 3 zu entnehmen. Bei den Komponenten handelt es sich um den präparierten Zahnstumpf 44, Schalen bzw. Schalenelemente 46, 48 zur Verfügung stellende Kontaktflächen der dem Zahnstumpf benachbarten Zähne sowie ein schalenförmiges Bauteil 50, das die Gegenbezahnungsgeometrie wiedergibt. Die entsprechenden Bauteile werden sodann entsprechend der digitalen 3D-Daten räumlich zueinander angeordnet, indem diese in der räumlichen Zuordnung berücksichtigenden Aufnahmen des ersten und zweiten Elementes 30, 34 angeordnet werden, die den Ober- bzw. Unterkiefer repräsentieren.

Ausgehend von dem zuvor erläuterten Minimalset aus den Schalenelementen 46, 48 und dem Zahnstumpf 44 kann die dargestellte Zahngeometrie bis zu einem Vollmodell erweitert werden, wenn z. B. ein Zahntechniker die Krümmungsinformation einer Buccalfläche der Nachbarzähne nutzen möchte.

Jedes der Elemente 44, 46, 48, 50 weist ein Verbindungselement 52, 54, 56, 58 wie Zapfen auf, um in die Aufnahmen der ersten und zweiten Elemente 30, 34 positionsgenau angeordnet werden zu können. Zur Aufnahme und Fixierung ist z. B. eine Loch/Pin-Anordnung geeignet, die im Ausschnitt der Fig. 3 zu entnehmen und mit dem Bezugszeichen 60 für den Unterkiefer und mit dem Bezugszeichen 62 für den Oberkiefer gekennzeichnet ist.

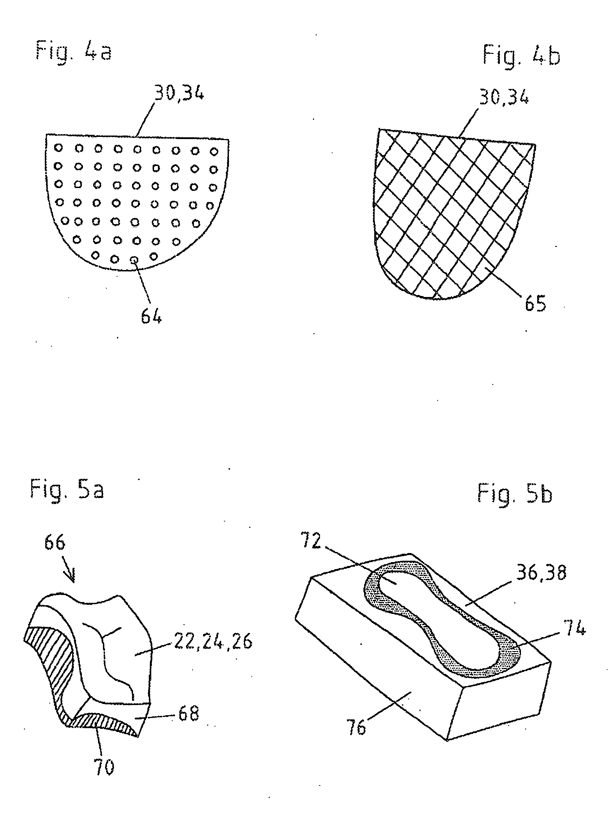

Wie die Fig. 4a und 4b verdeutlichen, sind die auch als Basisplatten zu bezeichnenden ersten und zweiten Elemente 30, 34 mit einem Loch/Pin Muster 64 oder ein Kreuzlinienmuster 65 wie Kreuznuten versehen, die Aufnahmen für die Verbindungselemente 52, 54, 56, 58 der Zahnelemente 44, 46, 48, 50 dienen. Dabei kann eine Verbindung über geeignete friktionsgehaltene Verzapfungskonzepte mit leichter Presspassung erfolgen.

Erfindungsgemäß besteht die Möglichkeit, dass die Bewegungsfelder 22, 24, 26 und die Bewegungsbegrenzer 36, 38 ergänzend gemessene Resilienzen nachbilden. Um dies konstruktiv umzusetzen, besteht die Möglichkeit, die die Bewegungsfelder 22, 24, 26 aufweisenden Bauteile materialmäßig entsprechend zu konzipieren. Dies ist anhand der Fig. 5a prinzipiell dargestellt. So besteht ein das Bewegungsfeld 22, 24, 26 als Oberfläche aufweisendes Bauteil 66 aus Materialschichten 68, 70, von denen die untere Schicht 70, von der das Bewegungsfeld 22, 24, 26 nicht ausgeht, eine Elastizität derart aufweist, dass beim Bewegen der Abstützelemente 16, 18, 20 auf den Bewegungsfeldern 22, 24, 26 im erforderlichen Umfang ein Nachgeben erfolgt, das der Resilienz entspricht.

Durch individuelle Schichtdicken oder Materialausweis, die aus den am Patienten gewonnenen Daten berechnet werden, lassen sich infolgedessen das individuelle Resilienzverhalten des Kiefergelenks bis hin zur Resilienz von Einzelzähnen darstellen.

Entsprechendes gilt für die Bewegungsbegrenzer 36, 38, die eine von den staubförmigen Abstützungen 18, 20 durchsetzte Aussparung 72 aufweisen, die von einer Schicht 74 gewünschter Elastizität begrenzt ist, um die ligamentäre und knorpelgeführte Begrenzung des Kiefergelenks nachzuempfinden. Die Schicht 74 wird sodann von einem Außenkörper 76 aufgenommen.

Aufgrund der erfindungsgemäßen Lehre und der Möglichkeit, mit individualisierten Minimalbauteilen Kieferbereiche nachzuempfinden, die mit einem Zahnersatz bestückt werden sollen, ergeben sich zu konventionellen Verfahren, bei denen ein vollständiges Modell von Unter- und Oberkiefer oder ein Quadrantenmodell benötigt wird, materialmäßig erhebliche Vorteile. Die Materialersparnis in Bezug auf die benötigte Höhe der Zahnteile beträgt im Vergleich zu einem Vollmodell 62 % und zu einem Quadrantenmodell 70 %. Die Volumen- und damit Materialersparnis beträgt im Vergleich zu einem Vollmodell 98 % und zu einem Quadrantenmodell 92 %.

**Bezugszeichenliste**

| | | | |
|---|---|---|---|
| 10 | Anordnung | 65 | Kreuzlinienmuster |
| 12 | Oberteil | 66 | Bauteil |
| 14 | Unterteil | 68 | Materialschicht |
| 16 | Abstützung | 70 | Materialschicht |
| 18 | Abstützung | 72 | Aussparung |
| 20 | Abstützung | 74 | Schicht |
| 22 | Bewegungsfeld | 76 | Außenkörper |
| 24 | Bewegungsfeld | | |
| 26 | Bewegungsfeld | | |
| 28 | Aufnahmeplatte | | |
| 30 | erstes Element | | |
| 32 | Aufnahmeplatte | | |
| 34 | zweites Element | | |
| 36 | Bewegungsbegrenzer | | |
| 38 | Bewegungsbegrenzer | | |
| 40 | Daten | | |
| 42 | Daten | | |
| 44 | Zahnstumpf | | |
| 46 | Schalenelement | | |
| 48 | Schalenelement | | |
| 50 | Bauteil | | |
| 52 | Verbindungselement | | |
| 54 | Verbindungselement | | |
| 56 | Verbindungselement | | |
| 58 | Verbindungselement | | |
| 60 | Loch/Pin-Anordnung | | |
| 62 | Loch/Pin-Anordnung | | |
| 64 | Loch/Pin-Anordnung | | |

## Patentansprüche

1. Verfahren zur Bildung eines physischen oder virtuellen Dentalmodells als Hilfsmittel für die Herstellung von Zahnersatz sowie dessen Herstellung, wobei Form zumindest eines in einem Ober- oder Unterkiefer vorhandenen Zahnstumpfs, Kontaktpunkte zu zu dem zumindest einen Zahnstumpf benachbarten Zähnen sowie Okklusalfläche zumindest eines Gegenzahns im Bereich des Bewegungsfeldes des auf dem zumindest einen Zahnstumpf anzuordnenden Zahnersatzes unter Verwendung digitaler Daten des berührungslos gemessenen Ober- und Unterkiefers berücksichtigt werden, wobei zumindest die Verfahrensschritte
a) Aufnehmen des Ober- und Unterkiefers zur Ennittlung erster Daten,
b) Aufnehmen des Bewegungsfeldes des Kiefergelenks in Disklusion zur Ermittlung zweiter Daten,
c) Aufnahme des Kontaktfeldes der Zähne in Okklusion in Zentrik und umfänglichem Bewegungsfeld zur Ermittlung dritter Daten,
d) Berechnung von Bewegungsbahnen zwischen Unter- und Oberkiefer aus zumindest den ersten Daten und den dritten Daten oder den ersten und den zweiten Daten oder den zweiten und den dritten Daten oder den ersten Daten und den zweiten Daten und den dritten Daten,
e) Verwenden einer Halteeinrichtung mit Unterteil und zu diesem verstellbarem Oberteil, wobei mit dem Oberteil ein den Oberkiefer repräsentierendes erstes Element mit ersten Aufnahmen und mit dem Unterteil ein den Unterkiefer repräsentierendes zweite Element mit zweiten Aufnahmen verbunden ist oder wird,
f) Herstellen von drei Abstützpunkten oder -flächen, die von den gemäß Schritt d) berechneten Bewegungsbahnen gebildet werden und die in ortsfester Beziehung zu dem Unterteil angeordnet werden oder sind und auf denen sich das Oberteil über Abstützelemente abstützt,
g) Herstellen des zumindest einen Stumpfs sowie zumindest einer zugewandten Seite der benachbarten Zähne sowie der Okklusionsfläche des zumindest einen Gegenzahns und Anordnung dieser in den ersten und zweiten Aufnahmen unter Berücksichtigung der ersten, zweiten und/oder dritten Daten und
h) Herstellen des Zahnersatzes auf dem zumindest einen Stumpf,
durchgeführt werden,
wobei die Verfahrensschritte b), c) alternativ oder sowohl der Verfahrensschritt b) als auch der Verfahrensschritt c) durchgeführt werden.

2. Verfahren nach Anspruch 1,
**dadurch gekennzeichnet,**
**dass** die Verfahrensschritte e), f), g) und h) virtuell durchgeführt werden.

3. Verfahren nach zumindest einem der vorhergehenden Ansprüche,
**dadurch gekennzeichnet,**
**dass** die Okklusion im Schritt c) mit therapeutischer Korrektur aufgenommen wird.

4. Verfahren nach zumindest einem der vorhergehenden Ansprüche,
**dadurch gekennzeichnet,**
**dass** die Okklusion im Verfahrensschritt c) zwangsgeführt durchgeführt wird.

5. Verfahren nach zumindest einem der vorhergehenden Ansprüche,
**dadurch gekennzeichnet,**
**dass** das Resilienzfeld des Kiefergelenks und/oder Mandibula und/oder Zahnapparat zur Ermittlung vierter Daten aufgenommen wird, wobei die Abstützpunkte oder -flächen unter Berücksichtigung der vierten Daten in ihrer Resilienz ausgebildet werden.

6. Verfahren nach zumindest einem der vorhergehenden Ansprüche,
**dadurch gekennzeichnet,**
**dass** den Abstützungen Bewegungsbegrenzungselemente zugeordnet werden oder sind, durch die die Bewegung des Oberteils zu dem Unterteil entsprechend der Kieferbewegung und/oder des gemessenen Resilienzfeldes des Kiefergelenks begrenzt wird, wobei vorzugsweise die Kieferbewegung entsprechend Verfahrensschritt b) ermittelt wird.

7. Verfahren nach zumindest einem der vorhergehenden Ansprüche,
**dadurch gekennzeichnet,**
**dass** ergänzend die Resilienz des Zahnhalteapparates von zumindest einem Zahn zur Ermittlung fünfter Daten gemessen wird, die zumindest beim Verfahrensschritt d) berücksichtigt werden.

8. Verfahren zur Bildung eines physischen oder virtuellen Dentalmodells als Hilfsmittel für die Herstellung von Zahnersatz sowie dessen Herstellung, wobei Form zumindest eines in einem Ober- oder Unterkiefer vorhandenen Zahnstumpfs, Kontaktpunkte zu zu dem zumindest einen Zahnstumpf benachbarten Zähnen sowie Okklusalfläche zumindest eines Gegenzahns im Bereich des Bewegungsfeldes des auf dem zumindest einen Zahnstumpf anzuordnenden Zahnersatzes unter Verwendung digitaler Daten des berührungslos gemessenen Ober- und Unterkiefers berücksichtigt werden, wobei zumindest die Verfahrensschritte
a) Aufnehmen des Ober- und Unterkiefers zur Ermittlung erster Daten,
b) Aufnehmen des Bewegungsfeldes des Kiefergelenks in Disklusion zur Ermittlung zweiter Daten,
c) Aufnahme der lateralen Bewegungen des Unterkiefers zum Oberkiefer unter Verwendung eines Verfahrens der minimalen Zunahme potentieller Energie bei den Bewegungen zur Ermittlung sechster Daten,
d) Berechnung von Bewegungsbahnen zwischen Unter- und Oberkiefer aus zumindest den ersten Daten und den sechsten Daten oder den ersten und den zweiten Daten oder den zweiten und den sechsten Daten oder den ersten Daten und den zweiten Daten und den sechsten Daten,
e) Verwenden einer Halteeinrichtung mit Unterteil und zu diesem verstellbarem Oberteil, wobei mit dem Oberteil ein den Oberkiefer repräsentierendes erstes Element mit ersten Aufnahmen und mit dem Unterteil ein den Unterkiefer repräsentierendes zweite Element mit zweiten Aufnahmen verbunden ist oder wird,
f) Herstellen von drei Abstützpunkten oder -flächen, die von den gemäß Schritt d) berechneten Bewegungsbahnen gebildet werden und die in ortsfester Beziehung zu dem Unterteil angeordnet werden oder sind und auf denen sich das Oberteil über Abstützelemente abstützt,
g) Herstellen des zumindest einen Stumpfs sowie zumindest einer zugewandten Seite der benachbarten Zähne sowie der Okklusionsfläche des zumindest einen Gegenzahns und Anordnung dieser in den ersten und zweiten Aufnahmen unter Berücksichtigung der ersten, zweiten und/oder dritten Daten und
h) Herstellen des Zahnersatzes auf dem zumindest einen Stumpf,
durchgeführt werden,
wobei die Verfahrensschritte b), c) alternativ oder sowohl der Verfahrensschritt b) als auch der Verfahrensschritt c) durchgeführt werden.

9. Verfahren nach zumindest Anspruch 1 oder 8,
**dadurch gekennzeichnet,**
**dass** die aus zumindest den ersten und dritten Daten oder alternativ sechsten Daten erfolgende Berechnung der Abstützpunkte oder -flächen gemäß Verfahrensschritt f) unter Reduktion der Freiheitsgrade der Beweglichkeit des Unterkiefers relativ zum Oberkiefer durchgeführt werden, wobei eine Rotation um die Sagittalachse (x-Achse), eine Rotation um die Transversalachse (y-Achse) und eine Translation entlang der z-Achse senkrecht zur Kauebene ausgeschlossen werden.

10. Verfahren nach zumindest Anspruch 8,
**dadurch gekennzeichnet,**
**dass** als Ausgangspunkt für die Berechnung der lateralen Bewegung Zentrik von Oberkiefer und Unterkiefer gewählt wird.

11. Verfahren nach zumindest einem der vorhergehenden Ansprüche,
**dadurch gekennzeichnet,**
**dass** zur Ermittlung der Zentrik von Unterkiefer und Oberkiefer buccale Seitenfläche der Zähne des Oberkiefers und Unterkiefers in Schlussbissstellung gescannt werden.

12. Verfahren nach zumindest einem der vorhergehenden Ansprüche,
**dadurch gekennzeichnet,**
**dass** die ersten, zweiten und dritten Daten intraoral ermittelt werden.

13. Anordnung (10) zur Herstellung eines Zahnersatzes unter Berücksichtigung diagnostischer Informationen eines Patienten, für den der Zahnersatz herzustellen ist, umfassend eine Halterung mit einem Unterteil (14) und einem zu diesem verstellbaren Oberteil (12), das über Abstützungen (16, 18, 20) auf drei im Bereich von Ecken eines Dreiecks liegenden Abstützflächen (22, 24, 26) gleitend abgestützt ist, die in ortsfester Beziehung zu dem Unterteil angeordnet sind oder von diesem ausgehen, wobei das Oberteil eine Halterung für ein einen Oberkiefer repräsentierendes erstes Element (30) mit ersten Aufnahmen und das Unterteil eine Halterung für ein einen Unterkiefer repräsentierendes zweites Element (34) mit zweiten Aufnahmen aufweist, wobei in den ersten und zweiten Aufnahmen zumindest ein mit dem Zahnersatz zu versehener Zahnstumpf (44), zumindest zugewandte Seiten von dem Zahnstumpf benachbarten Zähnen und Okklusalfläche zumindest eines Gegenzahns angeordnet sind, wobei die Abstützflächen Bewegungsinformationen von Unterkiefer und Oberkiefer in Zahnkontakt des Patienten enthalten und die Abstützflächen die Zuordnung der ersten und zweiten Aufnahmen und die Positionen des zumindest einen Zahnstumpfs sowie der zumindest zugewandten Seitenflächen und der Okklusalfläche auf der Basis von Daten berechnet sind, die mittels intraoraler Messung von Unter- und Oberkiefer des Patienten und zumindest der Stellung dieser in der Zentrik ermittelt werden, wobei die Abstützflächen (22, 24, 26) Oberflächen eines mit dem Unterteil verbundenen Körpers (66) sind, deren lokale Elastizitäten den auf die jeweiligen Abstützungspositionen umgerechneten Summenelastizitäten der Kiefergelenke und der Parodontien der jeweils im okklusalen Zahnkontakt stehenden Zahnpaare des Patienten entsprechen, und wobei zumindest zwei Abstützungen (16, 18, 20) des Oberteils beabstandet zu den Abstützflächen (22, 24, 26) von Begrenzungselementen (36, 38) umgeben sind, die die Bewegungsbegrenzung des Kiefers des Patienten berücksichtigen.

14. Anordnung nach Anspruch 13,
**dadurch gekennzeichnet,**
**dass** die Daten zumindest die dem Anspruch 1 zu entnehmenden ersten und dritten Daten oder ersten und zweiten Daten oder ersten, zweiten und dritten Daten sind, wobei die dritten Daten durch die sechsten Daten gemäß Anspruch 8 ersetzbar sind.

15. Anordnung nach Anspruch 13,
**dadurch gekennzeichnet,**
**dass** das Begrenzungselement (36, 38) eine die Resilienz des Kiefers berücksichtigende Elastizität aufweist.

16. Anordnung nach Anspruch 13,
**dadurch gekennzeichnet,**
**dass** die Seitenflächen der dem Stumpf (44) benachbarten Zähne und die Gegenbezahnung aus transparentem Material gefertigt werden, um die Lagen der Kontaktpunkte direkt beobachten zu können.

## Claims

1. A method for forming a physical or virtual dental model, as an aid in the manufacture of a dental prosthesis, as well as its manufacture, whereby the shape of at least one tooth stump present in an upper or lower jaw, contact points to teeth adjacent to the at least one tooth stump, as well as the occlusal surface of at least one antagonist tooth in the range of the field of motion of the dental prosthesis to be placed onto the at least one tooth stump are taken into consideration using digital data of the upper and lower jaw measured in a contact-less manner, whereby
at least the procedural steps
a) recording the upper and lower jaw to acquire first data,
b) recording the field of motion of the temporomandibular joint during disclusion to acquire second data,
c) recording the contact field of the teeth during occlusion in the centric position and ambient field of motion to acquire third data,
d) computing paths of motion between the upper and lower jaw from at least the first data and the third data, or the first data and the second data, or the second data and the third data, or the first data and the second data and the third data,
e) use of a holding device with a lower part and an upper part adjustable relative to the lower part, whereby connected to the upper part has been or is a first element, which represents the upper jaw and comprises first receptacles, and to the lower part has been or is being connected a second element, which represents the lower jaw and comprises second receptacles,
f) manufacturing of three support points or support surfaces, which are formed by the paths of motion computed pursuant to step d) and which have been or are being disposed stationary relative to the lower part and upon which the upper part is supported via supporting elements,
g) manufacturing the at least one stump as well as at least one facing side of the neighboring teeth as well as the occlusion surface of the at least one antagonist tooth and arranging these in the first and second receptacles, taking into consideration the first, second, and/or third data, and
h) manufacturing the dental prosthesis on the at least one stump,
are carried out,
whereby either the procedural step b) or the procedural step c) is performed, or both the procedural step b) and the procedural step c) are performed.

2. The method according to claim 1,
**characterized in that**
the procedural steps e), f), g) and h) are carried out in a virtual manner.

3. The method according to at least one of the previous claims,
**characterized in that**
the occlusion in step c) is recorded with a therapeutic correction.

4. The method according to at least one of the previous claims,
**characterized in that**
the occlusion in the procedural step c) is conducted in a forced manner.

5. The method according to at least one of the previous claims,
**characterized in that**
the resiliency field of the temporomandibular joint and/or mandible and/or dental apparatus is recorded to acquire fourth data, whereby the resiliency of the support points or surfaces is embodied taking into account the fourth data.

6. The method according to at least one of the previous claims,
**characterized in that**
motion limiter elements have been or are being associated with the supports, and are used to limit the movement of the upper part relative to the lower part, in accordance with the jaw movement and/or the measured resiliency field of the temporomandibular joint, whereby preferably the jaw movement is determined in accordance with procedural step b).

7. The method according to at least one of the previous claims,
**characterized in that**
additionally, the resiliency of the periodontium of at least one tooth is measured for the purpose of determining fifth data, which are taken into account at least in procedural step d).

8. A method for forming a physical or virtual dental model, as an aid in the manufacture of a dental prosthesis, as well as its manufacture, whereby the shape of at least one tooth stump present in an upper or lower jaw, contact points to teeth adjacent to the at least one tooth stump, as well as the occlusal surface of at least one antagonist tooth in the range of the field of motion of the dental prosthesis to be placed onto the at least one tooth stump are taken into consideration using digital data of the upper and lower jaw measured in a contact-less manner, whereby
at least the procedural steps
a) recording the upper and lower jaw to acquire first data,
b) recording the field of motion of the temporomandibular joint during disclusion to acquire second data,
c) recording the lateral motion of the lower jaw relative to the upper jaw using a minimum-increase-of-potential-energy method during the movements to determine sixth data,
d) computing paths of motion between the upper and lower jaw from at least the first data and the sixth data, or the first data and the second data, or the second data and the sixth data, or the first data and the second data and the sixth data,
e) use of a holding device with a lower part and an upper part adjustable relative to the lower part, whereby connected to the upper part has been or is a first element, which represents the upper jaw and comprises first receptacles, and to the lower part has been or is being connected a second element, which represents the lower jaw and comprises second receptacles,
f) manufacturing of three support points or support surfaces, which are formed by the paths of motion computed pursuant to step d) and which have been or are being disposed stationary relative to the lower part and upon which the upper part is supported via supporting elements,
g) manufacturing the at least one stump as well as at least one facing side of the neighboring teeth as well as the occlusion surface of the at least one antagonist tooth and arranging these in the first and second receptacles, taking into consideration the first, second, and/or third data, and
h) manufacturing the dental prosthesis on the at least one stump,
are carried out,
whereby
either the procedural step b) or the procedural step c) is performed, or both the procedural step b) and the procedural step c) are performed.

9. The method according to at least claim 1 or 8,
**characterized in that**
the computation of support points or support surfaces from at least the first or third data or alternatively from the sixth data in accordance with procedural step f) is performed by reducing the degrees of freedom of the movements of the lower jaw relative to the upper jaw, whereby a rotation about the sagittal axis (x axis), a rotation about the transverse axis (y axis), and a translation along the z axis perpendicular to the plane of occlusion are excluded.

10. The method according to at least claim 8,
**characterized in that**
as starting point for the computation of the lateral movement the centric position of upper jaw and lower jaw is chosen.

11. The method according to at least one of the previous claims,
**characterized in that**
for determining the centric position of the lower jaw and the upper jaw, the buccal side surfaces of the teeth of the upper jaw and the lower jaw are scanned in the terminal occlusal position.

12. The method according to one of the previous claims,
**characterized in that**
the first, second, and third data are acquired intra-orally.

13. An arrangement (10) for manufacturing a dental prosthesis taking into account diagnostic information about a patient for whom the prosthesis is to be manufactured, comprising a holding device with a lower part (14) and an upper part (12), which is adjustable relative to the lower part, is supported in a sliding manner via supports (16, 18, 20) on three support areas (22, 24, 26), which are situated in regions of the comers of a triangle and are arranged stationary relative to the lower part or originate from the lower part, whereby the upper part comprises a mount for a first element (30) representing the upper jaw with first receptacles and the lower part comprises a mount for a second element (34) representing the lower jaw with second receptacles, whereby in the first and the second receptacles at least one dental stump (44) to be provided with the dental prosthesis, at least the facing sides of the teeth adjacent to the tooth stump, and the occlusal surface of at least one antagonist tooth are arranged, whereby the support surfaces contain information on the movement of the lower and upper jaw during the patient's dental occlusion and the support surfaces, the assignment of the first and the second receptacles, and the positions of the at least one tooth stump as well as the at least facing lateral surfaces and the occlusal surface are computed on the basis of data, which are determined by means of intra-oral measurement of the patient's upper and lower jaw, and at least in their centric position, whereby the support surfaces (22, 24, 26) are surfaces of a body (66) connected to the lower part, with local elasticities that correspond to the sum elasticities of the temporomandibular joints and the parodontia of the respective tooth pairs of the patient that are in occlusal contact, said sum elasticities are converted into the respective support positions, whereby at least two supports (16, 18, 20) of the upper part are at some distance from the support surfaces (22, 24, 26) encompassed by motion limiter elements (36, 38) that take into account the limited movements of the patient's jaw.

14. The arrangement according to claim 13,
**characterized in that**
the data are at least the first and third data, or the first and second data, or the first, second, and third data described in claim 1, whereby the third data may be replaced by the sixth data according to claim 8.

15. The arrangement according to claim 13,
**characterized in that**
the limiter element (36, 38) possesses an elasticity that takes into account the resiliency of the jaw.

16. The arrangement according to claim 13,
**characterized in that**
the lateral surfaces of teeth adjacent to the stump (44) and the antagonist teeth are manufactured from transparent material, in order to be able to directly observe the positions of the contact points.

## Revendications

1. Procédé de formation d'un modèle dentaire physique ou virtuel en tant qu'aide à la fabrication d'une prothèse dentaire ainsi que sa fabrication, dans lequel sont pris en compte la forme d'au moins un moignon existant dans une mâchoire supérieure ou inférieure, des points de contact avec les dents voisines de l'au moins un moignon ainsi que des faces occlusales d'au moins une dent antagoniste dans la zone du champ de mouvement de la prothèse dentaire destinée à être posée sur l'au moins un moignon, au moyen de données numériques obtenues par mesure sans contact de la mâchoire supérieure et de la mâchoire inférieure, le procédé consistant à réaliser au moins les étapes suivantes
a) enregistrement de la mâchoire supérieure et de la mâchoire inférieure pour déterminer des premières données,
b) enregistrement du champ de mouvement de l'articulation temporo-maxillaire en désocclusion pour déterminer des deuxièmes données,
c) enregistrement de la surface de contact des dents en occlusion en centrique et dans le champ de mouvement périphérique pour déterminer des troisièmes données,
d) calcul de voies de mouvement entre mâchoire inférieure et mâchoire supérieure à partir d'au moins les premières données et les troisièmes données ou des premières et deuxièmes données ou des deuxièmes et troisièmes données ou des premières données et des deuxièmes données et des troisièmes données,
e) utilisation d'un dispositif de maintien avec partie inférieure et partie supérieure réglable par rapport à l'inférieure, sachant qu'à la partie supérieure est relié un premier élément avec premiers logements représentant la mâchoire supérieure et qu'à la partie inférieure est relié un deuxième élément avec deuxièmes logements représentant la mâchoire inférieure,
f) fabrication de trois points ou faces d'appui, qui sont formés par les voies de mouvement calculées selon l'étape d) et disposés en relation stationnaire avec la partie inférieure, et sur lesquels s'appuie la partie supérieure au moyen d'éléments d'appui,
g) fabrication de l'au moins un moignon ainsi que d'au moins un côté lui faisant face des dents voisines ainsi que de la face occlusale de l'au moins une dent antagoniste et placement de ceux-ci dans les premiers et deuxièmes logements en tenant compte des premières, deuxièmes et/ou troisièmes données, et
h) fabrication de la prothèse dentaire sur l'au moins un moignon, sachant que les étapes de procédé b), c) sont réalisées de façon alternative ou que l'étape de procédé b) ainsi que l'étape de procédé c) sont réalisées.

2. Procédé selon la revendication 1,
**caractérisé en ce**
**que** les étapes de procédé e), f), g) et h) sont réalisées de manière virtuelle.

3. Procédé selon au moins une des revendications précédentes,
**caractérisé en ce**
**que** l'occlusion dans l'étape c) est enregistrée avec correction thérapeutique.

4. Procédé selon au moins une des revendications précédentes,
**caractérisé en ce**
**que** l'occlusion dans l'étape de procédé c) est réalisée de manière forcée.

5. Procédé selon au moins une des revendications précédentes,
**caractérisé en ce**
**que** le champ de résilience de l'articulation temporo-maxillaire et/ou du maxillaire inférieur et/ou de l'appareil dentaire est enregistré pour déterminer des quatrièmes données, sachant que les points ou faces d'appui sont formés dans leur résilience en tenant compte des quatrièmes données.

6. Procédé selon au moins une des revendications précédentes,
**caractérisé en ce**
**qu'**aux appuis sont associés des éléments de limitation de mouvement, par lesquels est/sont limité(s) le mouvement de la partie supérieure par rapport à la partie inférieure correspondant au mouvement de la mâchoire et/ou du champ de résilience mesuré de l'articulation temporo-maxillaire, sachant qu'en particulier le mouvement de la mâchoire est déterminé conformément à l'étape de procédé b).

7. Procédé selon au moins une des revendications précédentes,
**caractérisé en ce**
**qu'**en complément est mesurée la résilience du parodonte d'au moins une dent pour déterminer des cinquièmes données qui sont prises en compte dans au moins l'étape de procédé d).

8. Procédé de formation d'un modèle dentaire physique ou virtuel en tant qu'aide à la fabrication d'une prothèse dentaire ainsi que sa fabrication, dans lequel sont pris en compte la forme d'au moins un moignon existant dans une mâchoire supérieure ou inférieure, des points de contact avec les dents voisines de l'au moins un moignon ainsi que des faces occlusales d'au moins une dent antagoniste dans la zone du champ de mouvement de la prothèse dentaire destinée à être posée sur l'au moins un moignon, au moyen de données numériques obtenues par mesure sans contact de la mâchoire supérieure et de la mâchoire inférieure, le procédé consistant à réaliser au moins les étapes suivantes
a) enregistrement de la mâchoire supérieure et de la mâchoire inférieure pour déterminer des premières données,
b) enregistrement du champ de mouvement de l'articulation temporo-maxillaire en désocclusion pour déterminer des deuxièmes données,
c) enregistrement des mouvements latéraux de la mâchoire inférieure par rapport à la mâchoire supérieure en mettant en oeuvre un procédé d'augmentation minimale d'énergie potentielle lors des mouvements pour déterminer des sixièmes données,
d) calcul de voies de mouvement entre mâchoire inférieure et mâchoire supérieure à partir d'au moins les premières données et les sixièmes données ou des premières et deuxièmes données ou des deuxièmes et des sixièmes données ou des premières données et des deuxièmes données et des sixièmes données,
e) utilisation d'un dispositif de maintien avec partie inférieure et partie supérieure réglable par rapport à l'inférieure, sachant qu'à la partie supérieure est relié un premier élément avec premiers logements représentant la mâchoire supérieure et qu'à la partie inférieure est relié un deuxième élément avec deuxièmes logements représentant la mâchoire inférieure,
f) fabrication de trois points ou faces d'appui, qui sont formés par les voies de mouvement calculées selon l'étape c) et disposés en relation stationnaire avec la partie inférieure et sur lesquels s'appuie la partie supérieure au moyen d'éléments d'appui,
g) fabrication de l'au moins un moignon ainsi que d'au moins un côté lui faisant face des dents voisines ainsi que de la face occlusale de l'au moins une dent antagoniste et placement de ceux-ci dans les premiers et deuxièmes logements en tenant compte des premières, deuxièmes et/ou troisièmes données, et
h) fabrication de la prothèse dentaire sur l'au moins un moignon,
sachant que les étapes de procédé b), c) sont réalisées de façon alternative ou que l'étape de procédé b) ainsi que l'étape de procédé c) sont réalisées.

9. Procédé selon au moins la revendication 1 ou 8,
**caractérisé en ce**
**que** le calcul des points ou faces d'appui selon l'étape de procédé f) obtenu à partir d'au moins les premières et troisièmes données ou en alternative des sixièmes données est effectué sous réduction du degré de liberté de la mobilité de la mâchoire inférieure relativement à la mâchoire supérieure, sachant qu'une rotation autour de l'axe sagittal (axe des x), une rotation autour de l'axe transversal (axe des y) et une translation le long de l'axe des z perpendiculairement au plan de mastication sont exclues.

10. Procédé selon au moins la revendication 8,
**caractérisé en ce**
**que** la centrique de la mâchoire supérieure et de la mâchoire inférieure est choisie comme base pour le calcul du mouvement latéral.

11. Procédé selon au moins une des revendications précédentes,
**caractérisé en ce**
**que** pour déterminer la centrique de la mâchoire inférieure et de la mâchoire supérieure, des faces latérales buccales des dents de la mâchoire supérieure et de la mâchoire inférieure sont scannées en position d'occlusion finale.

12. Procédé selon au moins une des revendications précédentes,
**caractérisé en ce**
**que** les premières, deuxièmes et troisièmes données sont déterminées de manière intra-orale.

13. Dispositif destiné à fabriquer une prothèse dentaire en tenant compte d'informations diagnostiques d'un patient pour lequel la prothèse dentaire, doit être fabriquée, comprenant un support avec une partie inférieure (14) et une partie supérieure (12) réglable par rapport à l'inférieure et qui par l'intermédiaire d'appuis (16, 18, 20) repose en pouvant glisser sur trois faces d'appui (22, 24, 26) situées dans la zone des angles d'un triangle et disposées en relation stationnaire avec la partie inférieure ou partant d'elle, sachant que la partie supérieure présente un support pour un premier élément (30) avec premiers logements représentant une mâchoire supérieure, et que la partie inférieure présente un support pour un deuxième élément (34) avec deuxièmes logements représentant une mâchoire inférieure, que dans les premiers et deuxièmes logements sont disposés au moins un moignon (44) devant être muni de la prothèse dentaire, au moins des côtés lui faisant face des dents voisines du moignon et des faces occlusales d'au moins une dent antagoniste, sachant que les faces d'appui contiennent des informations de mouvement de la mâchoire inférieure et de la mâchoire supérieure en contact dentaire du patient, et que les faces d'appui, l'affectation des premiers et deuxièmes logements, les positions de l'au moins un moignon, des au moins surfaces latérales lui faisant face ainsi que des faces occlusales sont calculées sur la base de données déterminées au moyen de mesure intra-orale des mâchoires inférieure et supérieure du patient et au moins de la position desdites mâchoires dans la centrique, sachant que les faces d'appui (22, 24, 26) sont les surfaces d'un corps (66) relié à la partie inférieure, dont les élasticités locales correspondent aux élasticités cumulées recalculées pour les positions des appuis respectifs de l'articulation temporo-maxillaire et des parodontes des paires de dents respectivement en contact occlusal du patient, et qu'au moins deux appuis (16, 18, 20) de la partie supérieure distants des faces d'appui (22, 24, 26) sont entourés par des éléments limiteurs (36, 38) qui prennent en compte la limitation de mouvement de la mâchoire du patient.

14. Dispositif selon la revendication 13,
**caractérisé en ce**
**que** les données sont au moins les premières et troisièmes données ou premières et deuxièmes données ou premières, deuxièmes et troisièmes données mentionnées dans la revendication 1, les troisièmes données pouvant être remplacées par les sixièmes données conformément à la revendication 8.

15. Dispositif selon la revendication 13,
**caractérisé en ce**
**que** l'élément limiteur (36, 38) présente une élasticité prenant en compte la résilience de la mâchoire.

16. Dispositif selon la revendication 13,
**caractérisé en ce**
**que** les surfaces latérales des dents voisines du moignon (44) et la denture antagoniste sont fabriquées dans un matériau transparent afin de pouvoir observer directement les positions des points de contact.
